# EUROPEAN PATENT SPECIFICATION

(11) **EP 3 876 828 B1**
(45) Date of publication and mention of the grant of the patent: **10.04.2024**
(21) Application number: 19836866.4
(22) Date of filing: 07.11.2019
(51) Int. Cl.: A61B 5/00, G16H 20/30, A63B 23/02, A61H 3/06, A61B 5/11, G16H 50/20, H04R 25/00, G09B 21/00

(54) **PHYSICAL THERAPY AND VESTIBULAR TRAINING SYSTEMS WITH VISUAL FEEDBACK**
PHYSIKALISCHE THERAPIE- UND VESTIBULÄRE TRAININGSYSTEME MIT VISUELLER RÜCKKOPPLUNG
SYSTÈMES DE THÉRAPIE PHYSIQUE ET D'ENTRAÎNEMENT VESTIBULAIRE AVEC RÉTROACTION VISUELLE

(30) Priority: 07.11.2018 US 201862756879 P
(43) Date of publication of application: 15.09.2021
(73) Proprietor: Starkey Laboratories, Inc., Eden Prairie, MN 55344 (US)
(72) Inventor: FABRY, David Alan, Eden Prairie, Minnesota 55347 (US); BHOWMIK, Achintya Kumar, Cupertino, California 95014 (US); BURWINKEL, Justin R., Eden Prairie, Minnesota 55347 (US); CRUKLEY, Jeffery Lee, Milton, Ontario L9T 0C5 (CA); SHAHAR, Amit, 4507329 Hod HaSharon (IL)
(74) Representative: Dentons UK and Middle East LLP
(86) International application number: PCT/US2019/060296
(87) International publication number: WO 2020/097353

(56) References cited:
- WO-A1-2018/127851
- US-A1- 2009 240 172
- US-A1- 2017 360 364
- US-B1- 9 414 784

## Description

This application is being filed as a PCT International Application on November 7, 2019 in the name of Starkey Laboratories, Inc., a U.S. national corporation, applicant for the designation of all countries and David Alan Fabry, a U.S. Citizen, and Achintya Kumar Bhowmik, a U.S. Citizen, and Justin R. Burwinkel, a U.S. Citizen, and Jeffery Lee Crukley, a Canadian Citizen, and Amit Shahar, an Israeli Citizen, inventors for the designation of all countries, and claims priority to U.S. Provisional Patent Application No. 62/756,879, filed November 7, 2018.

### Field

Embodiments herein relate to hearing assistance devices and related systems and methods for providing visual feedback to a subject undergoing physical therapy or vestibular movement training.

### Background

Each year, millions of patients visit a physician with complaints of dizziness. It is the most common complaint of patients over the age of 75, but it can occur in patients of any age. Dizziness is a general term that can be used to describe more specific feelings of unsteadiness, wooziness (swimming feeling in head), lightheadedness, feelings of passing out, sensations of moving, vertigo (feeling of spinning), floating, swaying, tilting, and whirling. Dizziness can be due to an inner ear disorder, a side effect of medications, a sign of neck dysfunction, or it can be due to a more serious problem such as a neurological or cardiovascular problem.

Conditions and symptoms related to dizziness can include imbalance, vertigo, Meniere's syndrome, benign paroxysmal positional vertigo (BPPV), vestibular neuritis, neck-related dizziness and migraines.

One approach to treating dizziness, imbalance, vertigo, Meniere's syndrome, benign paroxysmal positional vertigo (BPPV), neck-related dizziness and migraines is to have the patient perform exercises that can include vestibular rehabilitation exercises. Vestibular rehabilitation exercises are designed to improve balance and reduce problems related to dizziness. Beyond dizziness and the related conditions described above, vestibular rehabilitation may be used to treat patients who have had a stroke or brain injury or who have a propensity to fall.

WO 2018/127851 A1 relates to viewing apparatus such as virtual reality apparatus, and various methods therefor.

US 9,414,784 B1 relates to a movement assessment apparatus configured to provide biofeedback to a user regarding one or more movements executed by the user. The movement assessment apparatus generally includes a sensing device comprising one or more sensors, a data processing device operatively coupled to the sensing device, and a sensory output device operatively coupled to the data processing device. The data processing device is configured to determine executed motion data of a body portion of the user and/or executed motion data of an object manipulated by the user using one or more signals from the one or more sensors, to compare the executed motion data or a modified form of the executed motion data to baseline motion data of a reference motion, and to determine how closely the executed motion data or the modified form of the executed motion data conforms to the baseline motion data of the reference motion.

US2017/360364 discloses a hearing assistance device according to the preamble of claim 12.

### Summary

Embodiments herein relate to hearing assistance devices and related systems and methods for providing visual feedback to a subject undergoing physical therapy or vestibular movement training. In an embodiment, a method of providing visual feedback to a subject undergoing physical therapy or vestibular movement is included. The method includes providing a visual and/or auditory notification to the subject for moving in a predetermined direction by a predetermined amount as part of a first predetermined movement. The method further includes tracking movement of the subject using a first inertia measurement unit 'IMU' mounted in a first hearing assistance device and disposed in a fixed position relative to the subject's head. The method further includes generating feedback data by comparing the tracked movement of the subject against the predetermined direction and amount of the first predetermined movement. The method further includes generating a feedback image using the feedback data.

In an embodiment, a method of providing visual feedback to a subject undergoing physical therapy or vestibular movement is included. The method includes providing a visual and/or auditory notification to the subject for moving in a predetermined direction as part of a first predetermined movement. The method further includes tracking movement of the subject using an inertia measurement unit 'IMU' mounted in a first hearing assistance device and positioned in a fixed position relative to the subject's head. The method further includes generating a feedback image including a visual representation of the first predetermined movement and a visual representation of the tracked movement.

In an embodiment, a hearing assistance device is included. The device includes a first control circuit and a first inertia measurement unit IMU in electrical communication with the first control circuit. The first IMU is mounted in the hearing assistance device and disposed in a fixed position relative to a head of a subject wearing the hearing assistance device. The device also includes a first microphone in electrical communication with the first control circuit, a first electroacoustic transducer for generating sound in electrical communication with the first control circuit, and a first power supply circuit in electrical communication with the first control circuit. The first control circuit is configured to track movement of the subject using the first IMU and generate feedback data reflecting a comparison of the tracked movement of the subject against a predetermined direction and amount of movement as part of a first predetermined movement.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### Brief Description of the Figures

Aspects may be more completely understood in connection with the following figures (FIGS.), in which:
FIG. 1 is a partial cross-sectional view of ear anatomy.
FIG. 2 is a schematic view of a hearing assistance device in accordance with various embodiments herein.
FIG. 3 is a schematic view of various components of a hearing assistance device in accordance with various embodiments herein.
FIG. 4 is a schematic view of a hearing assistance device disposed within the ear of a subject in accordance with various embodiments herein.
FIG. 5 is a schematic view of data flow as part of a system in accordance with various embodiments herein.
FIG. 6 is a schematic side view of a subject wearing a hearing assistance device in accordance with various embodiments herein.
FIG. 7 is a schematic top view of a subject wearing hearing assistance devices in accordance with various embodiments herein.
FIG. 8 is a schematic view of a subject wearing a hearing assistance device and receiving visual feedback from an external visual display device in accordance with various embodiments herein.
FIG. 9 is a schematic view of an external visual display device and elements of visual display thereof.
FIG. 10 is a schematic view of a subject wearing a hearing assistance device in a standing posture in accordance with various embodiments herein.
FIG. 11 is a schematic view of a subject wearing a hearing assistance device in a supine posture in accordance with various embodiments herein.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, equivalents, and alternatives falling within the spirit and scope herein.

### Detailed Description

Exercises such as vestibular rehabilitation exercises can be useful for patients experiencing dizziness, imbalance, vertigo, Meniere's syndrome, benign paroxysmal positional vertigo (BPPV), neck-related dizziness and migraines, and the like. However, like all exercises, vestibular rehabilitation exercises are only useful if they are actually performed. In addition, some of these exercises can be difficult to perform properly without expert guidance.

Embodiments herein include hearing assistance devices and related systems and methods for providing visual feedback to a subject undergoing vestibular movement training exercises. This visual feedback can assist the subject in performing the exercises properly. In addition, embodiments herein can include providing data regarding performance of the exercises (or non-performance) to care providers and can convey feedback from care providers back to the subject.

The term "hearing assistance device" as used herein shall refer to devices that can aid a person with impaired hearing. The term "hearing assistance device" shall also refer to devices that can produce optimized or processed sound for persons with normal hearing. Hearing assistance devices herein can include hearables (e.g., wearable earphones, headphones, earbuds, virtual reality headsets), hearing aids (e.g., hearing instruments), cochlear implants, and bone-conduction devices, for example. Hearing assistance devices include, but are not limited to, behind-the-ear (BTE), in-the ear (ITE), in-the-canal (ITC), invisible-in-canal (IIC), receiver-in-canal (RIC), receiver in-the-ear (RITE) or completely-in-the-canal (CIC) type hearing assistance devices or some combination of the above.

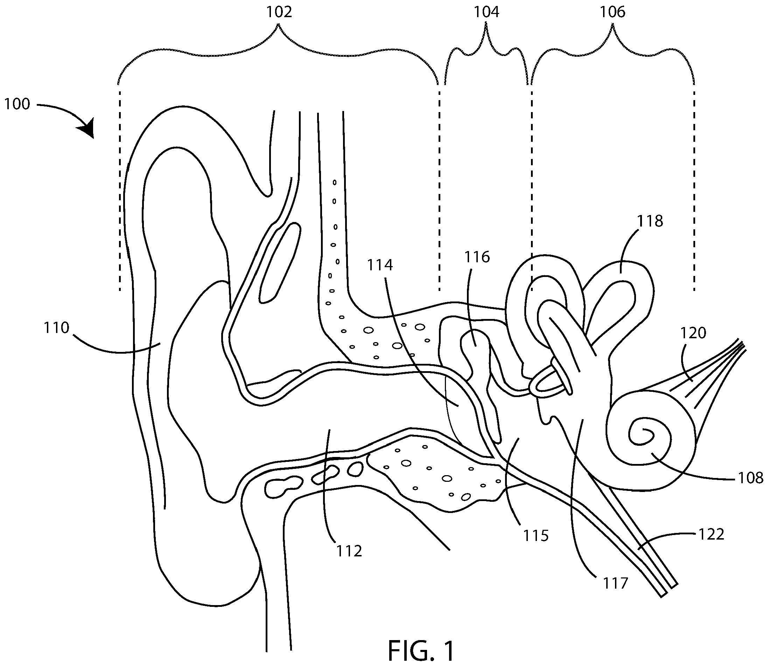

Referring now to FIG. 1, a partial cross-sectional view of ear anatomy 100 is shown. The three parts of the ear anatomy 100 are the outer ear 102, the middle ear 104 and the inner ear 106. The outer ear 102 includes the pinna 110, ear canal 112, and the tympanic membrane 114 (or eardrum). The middle ear 104 includes the tympanic cavity 115 and auditory bones 116 (malleus, incus, stapes). The inner ear 106 includes the cochlea 108, vestibule 117, semicircular canals 118, and auditory nerve 120. "Cochlea" means "snail" in Latin; the cochlea gets its name from its distinctive coiled up shape. The pharyngotympanic tube 122 is in fluid communication with the eustachian tube and helps to control pressure within the middle ear generally making it equal with ambient air pressure.

Sound waves enter the ear canal 112 and make the tympanic membrane 114 vibrate. This action moves the tiny chain of auditory bones 116 (ossicles - malleus, incus, stapes) in the middle ear 104. The last bone in this chain contacts the membrane window of the cochlea 108 and makes the fluid in the cochlea 108 move. The fluid movement then triggers a response in the auditory nerve 120.

Hearing assistance devices, such as hearing aids and hearables (e.g., wearable earphones), can include an enclosure, such as a housing or shell, within which internal components are disposed. Components of a hearing assistance device herein can include a control circuit, digital signal processor (DSP), memory (such as non-volatile memory), power management circuitry, a data communications bus, one or more communication devices (e.g., a radio, a near-field magnetic induction device), one or more antennas, one or more microphones, a receiver/speaker, and various sensors as described in greater detail below. More advanced hearing assistance devices can incorporate a long-range communication device, such as a Bluetooth^{®} transceiver or other type of radio frequency (RF) transceiver.

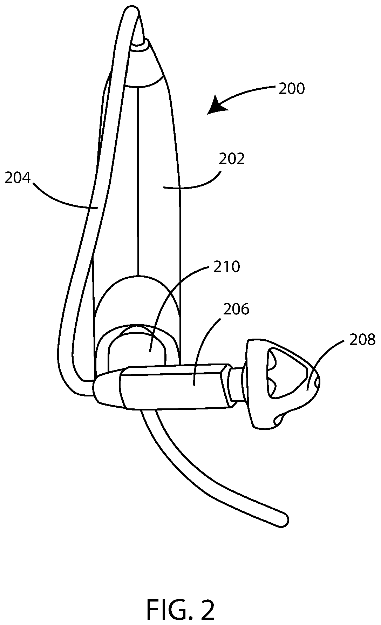

Referring now to FIG. 2, a schematic view of a hearing assistance device 200 is shown in accordance with various embodiments herein. The hearing assistance device 200 can include a hearing device housing 202. The hearing device housing 202 can define a battery compartment 210 into which a battery can be disposed to provide power to the device. The hearing assistance device 200 can also include a receiver 206 adjacent to an earbud 208. The receiver 206 an include a component that converts electrical impulses into sound, such as an electroacoustic transducer, speaker, or loud speaker. A cable 204 or connecting wire can include one or more electrical conductors and provide electrical communication between components inside of the hearing device housing 202 and components inside of the receiver 206.

The hearing assistance device 200 shown in FIG. 2 is a receiver-in-canal type device and thus the receiver is designed to be placed within the ear canal. However, it will be appreciated that may different form factors for hearing assistance devices are contemplated herein. As such, hearing assistance devices herein can include, but are not limited to, behind-the-ear (BTE), in-the ear (ITE), in-the-canal (ITC), invisible-in-canal (IIC), receiver-in-canal (RIC), receiver in-the-ear (RITE) and completely-in-the-canal (CIC) type hearing assistance devices.

Hearing assistance devices of the present disclosure can incorporate an antenna arrangement coupled to a high-frequency radio, such as a 2.4 GHz radio. The radio can conform to an IEEE 802.11 (e.g., WIFI^{®}) or BLUETOOTH^{®} (e.g., BLE, Bluetooth^{®} 4. 2 or 5.0) specification, for example. It is understood that hearing assistance devices of the present disclosure can employ other radios, such as a 900 MHz radio. Hearing assistance devices of the present disclosure can be configured to receive streaming audio (e.g., digital audio data or files) from an electronic or digital source. Representative electronic/digital sources (also referred to herein as accessory devices) include an assistive listening system, a TV streamer, a radio, a smartphone, a cell phone/entertainment device (CPED) or other electronic device that serves as a source of digital audio data or files.

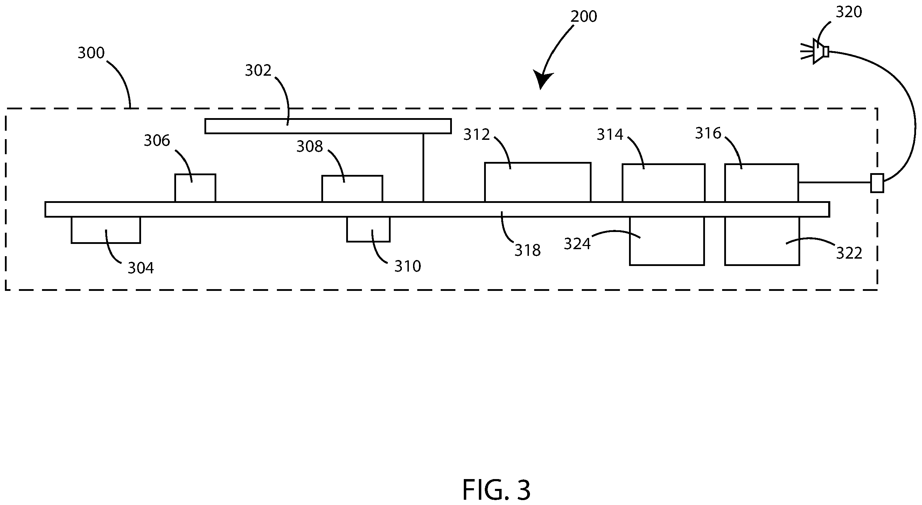

Referring now to FIG. 3, a schematic block diagram is shown with various components of a hearing assistance device in accordance with various embodiments. The block diagram of Figure 3 represents a generic hearing assistance device for purposes of illustration. The hearing assistance device 200 shown in FIG. 3 includes several components electrically connected to a flexible mother circuit 318 (e.g., flexible mother board) which is disposed within housing 300. A power supply circuit 304 can include a battery and can be electrically connected to the flexible mother circuit 318 and provides power to the various components of the hearing assistance device 200. One or more microphones 306 are electrically connected to the flexible mother circuit 318, which provides electrical communication between the microphones 306 and a digital signal processor (DSP) 312. Among other components, the DSP 312 incorporates or is coupled to audio signal processing circuitry configured to implement various functions described herein. A sensor package 314 can be coupled to the DSP 312 via the flexible mother circuit 318. The sensor package 314 can include one or more different specific types of sensors such as those described in greater detail below. One or more user switches 310 (e.g., on/off, volume, mic directional settings) are electrically coupled to the DSP 312 via the flexible mother circuit 318.

An audio output device 316 is electrically connected to the DSP 312 via the flexible mother circuit 318. In some embodiments, the audio output device 316 comprises a speaker (coupled to an amplifier). In other embodiments, the audio output device 316 comprises an amplifier coupled to an external receiver 320 adapted for positioning within an ear of a wearer. The external receiver 320 can include an electroacoustic transducer, speaker, or loud speaker. The hearing assistance device 200 may incorporate a communication device 308 coupled to the flexible mother circuit 318 and to an antenna 302 directly or indirectly via the flexible mother circuit 318. The communication device 308 can be a BLUETOOTH^{®} transceiver, such as a BLE (BLUETOOTH^{®} low energy) transceiver or other transceiver (e.g., an IEEE 802.11 compliant device). The communication device 308 can be configured to communicate with one or more external devices, such as those discussed previously, in accordance with various embodiments. In various embodiments, the communication device 308 can be configured to communicate with an external visual display device such as a smart phone, a video monitor, a video display screen, a smart mirror, a virtual reality device, an augmented reality device, a hologram generator, a tablet, a computer, or the like.

In various embodiments, the hearing assistance device 200 can also include a control circuit 322 and a memory storage device 324. The control circuit 322 can be in electrical communication with other components of the device. The control circuit 322 can execute various operations, such as those described herein. The control circuit 322 can include various components including, but not limited to, a microprocessor, a microcontroller, an FPGA (field-programmable gate array) processing device, an ASIC (application specific integrated circuit), or the like. The memory storage device 324 can include both volatile and non-volatile memory. The memory storage device 324 can include ROM, RAM, flash memory, EEPROM, SSD devices, NAND chips, and the like. The memory storage device 324 can be used to store data from sensors as described herein and/or processed data generated using data from sensors as described herein, including, but not limited to, information regarding exercise regimens, performance of the same, visual feedback regarding exercises, and the like.

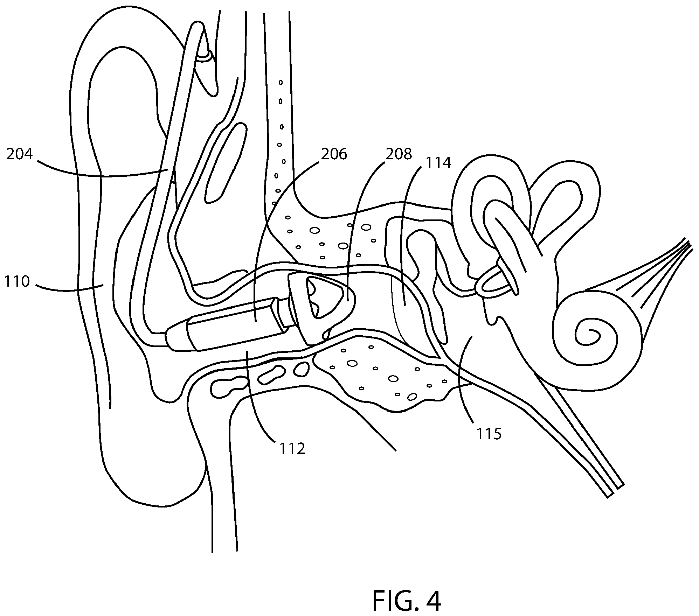

As mentioned with regard to FIG. 2, the hearing assistance device 200 shown in FIG. 2 is a receiver-in-canal type device and thus the receiver is designed to be placed within the ear canal. Referring now to FIG. 4, a schematic view is shown of a hearing assistance device disposed within the ear of a subject in accordance with various embodiments herein. In this view, the receiver 206 and the earbud 208 are both within the ear canal 112, but do not directly contact the tympanic membrane 114. The hearing device housing is mostly obscured in this view behind the pinna 110, but it can be seen that the cable 204 passes over the top of the pinna 110 and down to the entrance to the ear canal 112.

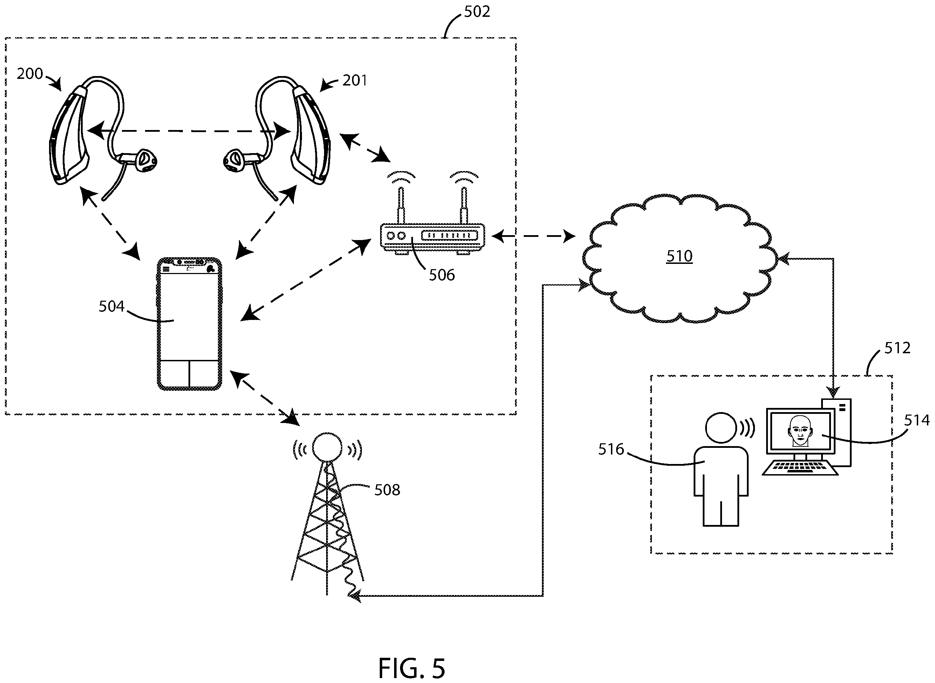

It will be appreciated that data and/or signals can be exchanged between many different components in accordance with embodiments herein. Referring now to FIG. 5, a schematic view is shown of data and/or signal flow as part of a system in accordance with various embodiments herein. In a first location 502, a user (not shown) can have a first hearing assistance device 200 and a second hearing assistance device 201. Each of the hearing assistance devices 200, 201 can include sensor packages as described herein including, for example, an IMU. The hearing assistance devices 200, 201 and sensors therein can be disposed on opposing lateral sides of the subject's head. The hearing assistance devices 200, 201 and sensors therein can be disposed in a fixed position relative to the subject's head. The hearing assistance devices 200, 201 and sensors therein can be disposed within opposing ear canals of the subject. The hearing assistance devices 200, 201 and sensors therein can be disposed on or in opposing ears of the subject. The hearing assistance devices 200, 201 and sensors therein can be spaced apart from one another by a distance of at least 3, 4, 5, 6, 8, 10, 12, 14, or 16 centimeters and less than 40, 30, 28, 26, 24, 22, 20 or 18 centimeters, or by a distance falling within a range between any of the foregoing.

In various embodiments, data and/or signals can be exchanged directly between the first hearing assistance device 200 and the second hearing assistance device 201. An external visual display device 504 with a video display screen, such as a smart phone, can also be disposed within the first location 502. The external visual display device 504 can exchange data and/or signals with one or both of the first hearing assistance device 200 and the second hearing assistance device 201 and/or with an accessory to the hearing assistance devices (e.g., a remote microphone, a remote control, a phone streamer, etc.). The external visual display device 504 can also exchange data across a data network to the cloud 510, such as through a wireless signal connecting with a local gateway device, such as a network router 506 or through a wireless signal connecting with a cell tower 508 or similar communications tower. In some embodiments, the external visual display device can also connect to a data network to provide communication to the cloud 510 through a direct wired connection.

In some embodiments, a care provider 516 (such as an audiologist, physical therapist, a physician or a different type of clinician, specialist, or care provider, or physical trainer) can receive information from devices at the first location 502 remotely at a second location 512 through a data communication network such as that represented by the cloud 510. The care provider 516 can use a computing device 514 to see and interact with the information received. The received information can include, but is not limited to, information regarding the subject's performance of the exercise including, but not limited to, whether or not exercises were performed, accuracy of exercise performance, time spent performing exercises, range of motion, and spatial position information related to IMU and/or accelerometer data, trends related to exercise performance (consistency, accuracy, etc.) and the like. In some embodiments, received information can be provided to the care provider 516 in real time. In some embodiments, received information can be stored and provided to the care provider 516 at a time point after exercises are performed by the subject.

In some embodiments, the care provider 516 (such as an audiologist, physical therapist, a physician or a different type of clinician, specialist, or care provider, or physical trainer) can send information remotely from the second location 512 through a data communication network such as that represented by the cloud 510 to devices at the first location 502. For example, the care provider 516 can enter information into the computing device 514, can use a camera connected to the computing device 514 and/or can speak into the external computing device. The sent information can include, but is not limited to, feedback information, guidance information, future exercise directions/regimens, and the like. In some embodiments, feedback information from the care provider 516 can be provided to the subject in real time. In some embodiments, received information can be stored and provided to the subject at a time point after exercises are performed by the subject or during the next exercise session that the subject performs.

As such, embodiments herein can include operations of sending the feedback data to a remote system user at a remote site, receiving feedback (such as auditory feedback) from the remote system user, and presenting the feedback to the subject. The operation of presenting the auditory feedback to the subject can be performed with the hearing assistance device(s). In various embodiments, the operation of presenting the auditory feedback to the subject can be performed with a hearing assistance device(s) and the auditory feedback can be configured to be presented to the subject as spatially originating (such as with a virtual audio interface described below) from a direction of an end point of the first predetermined movement.

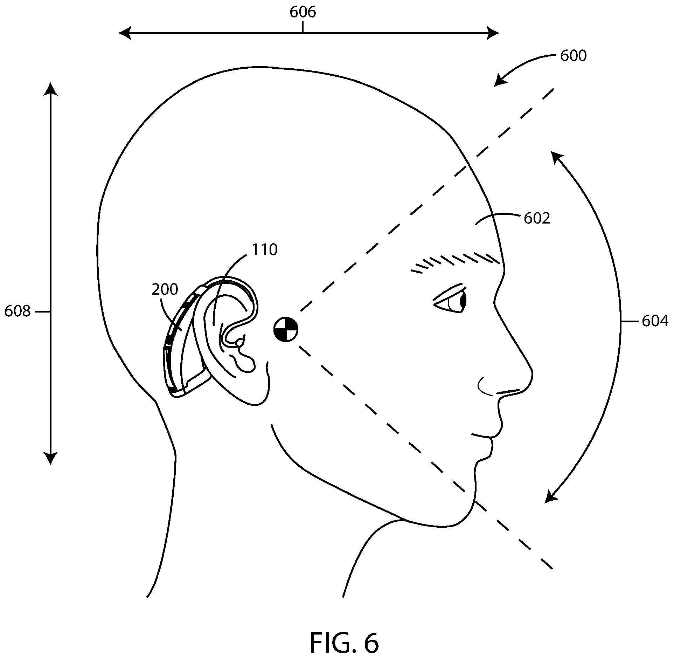

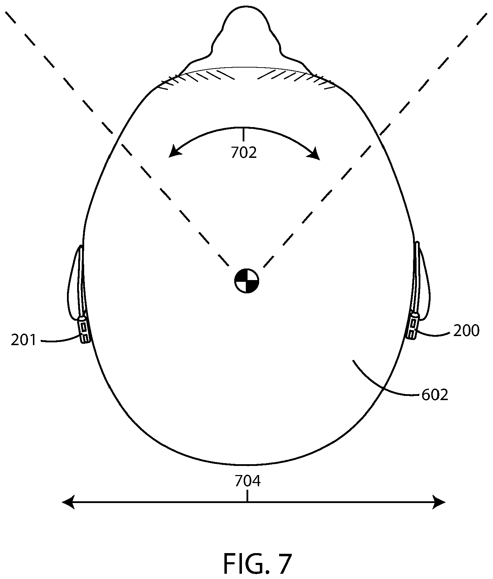

Hearing assistance devices herein can include sensors (such as part of a sensor package 314) to detect movements of the subject wearing the hearing assistance device. Referring now to FIG. 6, a schematic side view is shown of a subject 600 wearing a hearing assistance device 200 in accordance with various embodiments herein. For example, movements detected can include forward/back movements 606, up/down movements 608, and rotational movements 604 in the vertical plane. Referring now to FIG. 7, a schematic top view is shown of a subject 600 wearing hearing assistance devices 200, 201 in accordance with various embodiments herein. Movements detected can also include side-to-side movements 704, and rotational movements 702 in the horizontal plane.

In accordance with various embodiments herein, the hearing assistance device and/or the system can prompt the subject to move in a predetermined direction by a predetermined amount as part of a first predetermined movement, track movement of the subject using a first IMU or other sensor herein disposed in a fixed position relative to the subject's head, and generate feedback data by comparing the tracked movement of the subject against the predetermined direction and amount and generating a feedback image using the feedback data.

In some embodiments, prompts can be queued according to a schedule but not actually delivered to the subject (via a visual and/or an auditory notification) until one or more specific events are detected or a particular absence of one or more events is detected. By way of example, in some embodiments, the system and/or devices thereof can first queue the prompt according to a predetermined schedule and then trigger delivery of the prompt after detecting sedentary behavior of the subject. In some embodiments, the system and/or devices thereof can first queue the prompt according to a predetermined schedule and then trigger delivery of the prompt after detecting sedentary behavior of the subject, if the sedentary behavior is detected during a predefined time window, such as a normal awake period. Sedentary behavior can be detected in various ways including, but not limited to, accelerometer data that crosses a threshold value, heart rate data that crosses a threshold value, blood pressure data that crosses a threshold value, or the like. In some embodiments, prompting the subject can be performed if nystagmus is detected in the subject.

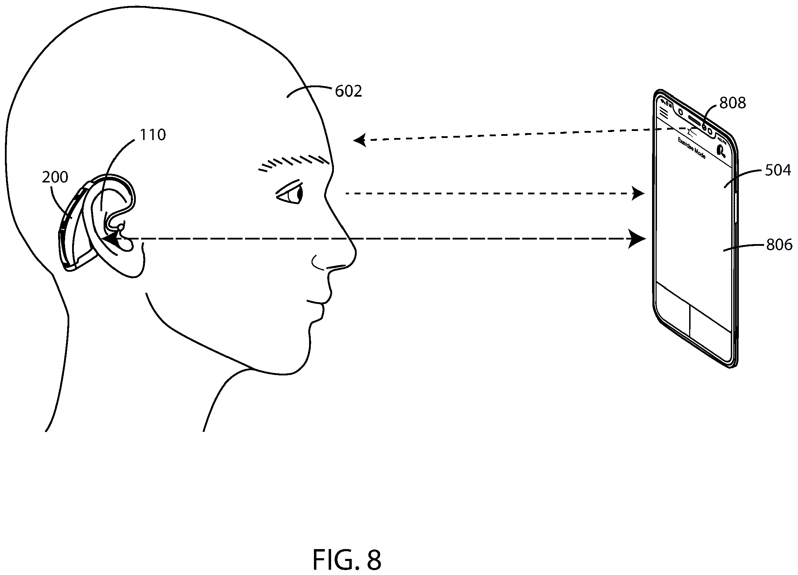

Referring now to FIG. 8, a schematic view is shown of a subject 602 wearing a hearing assistance device 200 and receiving visual feedback from an external visual display device 504 in accordance with various embodiments herein. The external visual display device 504 can include a display screen 806 and a camera 808. In some embodiments, the external visual display device 504 can be a smart phone, a video monitor, a video display screen, a smart mirror, a virtual reality device, an augmented reality device, a hologram generator, a tablet, a computer, or the like.

In some embodiments, the display screen 806 can be a touch screen. The display screen 806 can display various pieces of information to the subject 602 including, but not limited to, instructions for exercises, visual feedback regarding the fidelity with which the subject 602 is performing the exercises, a silhouette illustrating the position the patient's head needs to be moved to in order to complete the next step of the exercise, information regarding the progress of the subject 602 through a particular set of exercises, the remaining time to complete a particular set of exercises, current feedback from a care provider (remote or local), or the like. In some embodiments, positional guidance silhouettes can be presented as having two dimensions. However, in some embodiments, positional guidance silhouettes can be presented as having three dimensions and/or as a visually superposed image to provide an augmented reality within the user's environment or training space. In some embodiments, the display screen 806 can display an animation demonstrating how to perform the exercise, a visual counter of how many repetitions the user has completed, and, in some cases, one or more components of the system can provide audio feedback regarding the same. The system can be configured so that the subject 602 can begin an exercise using voice commands when they are ready.

The camera 808 can be positioned to face away from the display screen 806 and back toward the subject 602. The camera 808 can be used to capture an image or images of the subject's 602 face and, in some cases, the subject's 602 eyes.

In some embodiments, the camera 808 (or another camera) can be used to monitor movement of body parts including those other than the head, face, or eyes. For example a camera can be used to monitor movement of the legs or feet of the subject 602. In one example, the user can be seated and looking downward at the device, in which case a front facing camera can monitor the face/eyes of the user and a rear facing camera can monitor the legs/feet of the subject 602. Various other embodiments are contemplated for monitoring the body movements of the user.

In some embodiments, the camera 808 can be used to capture image(s) including the positioning of subject's 602 face, pupil, iris, and/or sclera. Such information can be used to calculate the direction of the subject's 602 face and/or gaze. In some embodiments, such information can also be used to calculate angle, speed and direction of nystagmus. Aspects of nystagmus detection and characterization are described in commonly-owned U.S. Publ. Pat. Appl. No. 2018/0228404, the content of which is herein incorporated by reference. In some embodiments, such information can specifically be used to calculate the direction of the subject's 602 face and/or gaze with respect to the camera 808. Aspects regarding such calculations are described in U.S. Publ. Appl. Nos. 2012/0219180 and 2014/0002586; the content of which is herein incorporated by reference.

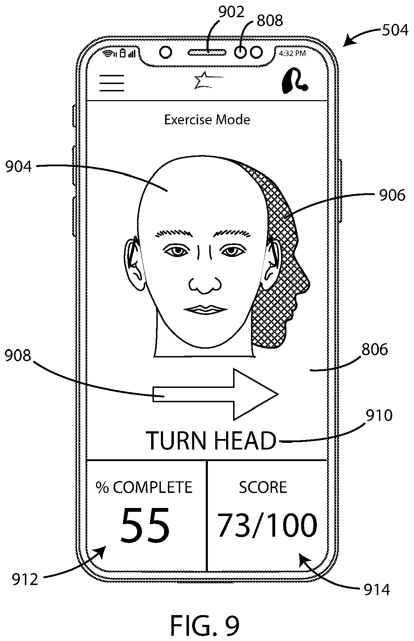

Referring now to FIG. 9, a schematic view is shown of an external visual display device 504 and elements of the display screen 806 thereof. The external visual display device 504 can include a camera 808, and a speaker 902. The external visual display device 504 can generate and/or display a feedback image using feedback data transmitted from one or two hearing assistance devices. In some embodiments, the feedback image is generated by one or two of the hearing assistance devices and then wirelessly transferred to the external visual display device 504 for display. In some embodiments, data from an operatively connected camera (which could be camera 808, or another camera that could be part of a separate device) can be used in combination with feedback data as sensed and/or transmitted from one or two hearing assistance devices.

The external visual display device 504 can display (or be operatively connected to a device that can display) a feedback image including a visual representation of the first predetermined movement and/or a visual representation of the tracked movement.

The visual representation of the first predetermined movement can include display of a head. The visual representation of the first predetermined movement can include a silhouette 906 of a head. The visual representation of the predetermined movement can change based on the feedback data. For example, the visual representation of the predetermined movement can change color based on the feedback data. In some embodiments, the size and/or perspective 906 of the silhouette can change, such as if the subject needs to move their head forward or backward, the size or perspective of the silhouette can change to guide them.

The visual representation of the tracked movement can include an image of the subject's head 904. The visual representation of the tracked movement can specifically include an image of the subject's head 904 reflecting the current position of the subject's head 904. Images of the subject's head 904 can be real (such as extracted from data provided by the camera 808) or can be a model image representing the subject's head 904.

The external visual display device 504 can display a feedback image including a directional icon 908, such as an arrow, indicating the direction that the patient should be moving their head. The directional icon can be provided as a mirror image so that the arrow can be directly followed in order to result in the proper movement of the patient's head (e.g., if the patient currently needs to rotate their head to the right in order to follow the determined movement of the exercise the arrow on the external visual display device 504 can be pointing to the left side of the screen as judged from the perspective of the external visual display device facing back toward the subject).

In various embodiments, the external visual display device 504 can display a feedback image including a textual instruction 910 guiding the subject to perform the determined movement of the exercise, such as "Turn Head" or "Turn Head 90° Right".

Various other pieces of data regarding the exercise or movements thereof can be displayed on the external visual display device 504. For example, information regarding the state of completion 912 of the exercise can be displayed on the external visual display device 504. Such state of completion 912 information can be displayed in the form of a current percent of completion of the exercise session, an elapsed time of the exercise session so far, a remaining time of the exercise session, or the like.

Information regarding the accuracy of the patient's performance of the exercise 914 can also be displayed on the external visual display device 504 and auditorily via the hearing assistance device 200. In some embodiments, the accuracy of the patient's performance of the exercise 914 can be displayed and reflected as a calculated score. By way of example, the score can be calculated based on percentages of accuracy of movements. If the current movement involves rotating the head 90 degrees to the right and the user only rotates their head 75 degrees, then the accuracy score for that movement could be 75/90, 83% or 83/100. The accuracy of the patient's performance of the exercise 914 shown on the external visual display device 504 can reflect an average of accuracy scores for each movement performed so far during the current exercise session. In various embodiments, the accuracy of the patient's performance of the exercise 914 shown on the external visual display device 504 can change visually based on the current degree of accuracy. For example, current scores or average scores above 90 can be shown in blue or green and scores below 50 can be shown in red. Many visual display options are contemplated herein.

### Estimating Body Position

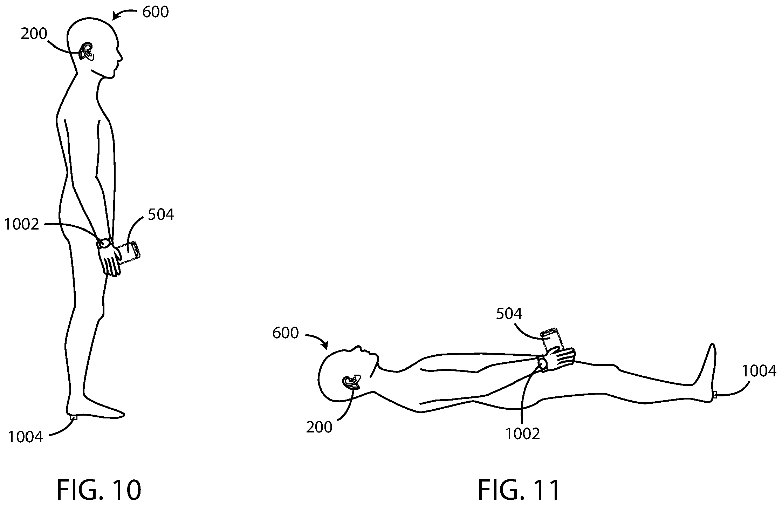

In accordance with various embodiments herein, the position of the subject's head can be determined. However, in some embodiments, the body position and/or posture of the subject can also be determined (or otherwise estimated or calculated). Referring now to FIG. 10, a schematic view of a subject 600 wearing a hearing assistance device 200 in a standing posture is shown in accordance with various embodiments herein. In this view, the subject 600 also is wearing a wearable device 1002, that can include circuitry and, specifically, a sensor package like those previously described for the hearing assistance device. In some embodiments, the subject 600 could also have hearing assistance device accessories such as a remote microphone, a remote control, a phone streamer, or the like. The wearable device 1002 can be in wireless data communication with the hearing assistance device 200. In this view, the subject 600 is holding onto an external visual display device 504, such as a smartphone. The external visual display device 504 can be in wireless data communication with the hearing assistance device 200. In this view, the subject 600 is standing on a weight or load sensor 1004, which can be a pressure sensor, force sensor, piezoelectric sensor or the like. In some embodiments, the weight or load sensor 1004 can be integrated into footwear, floor tiles, pads, scales or the like (not shown in this view). The weight or load sensor 1004 can be in wireless data communication with the hearing assistance device 200. FIG. 11 is like FIG. 10 in that it is a schematic view of a subject 600 wearing a hearing assistance device 200. However, in FIG. 11, the subject 600 is in a supine posture instead of a standing posture. While only standing and supine postures are shown in these two figures, various other positions/postures are contemplated herein including, sitting, prone, squatting, lying on side, etc.

As described previously, the hearing assistance device 200 can include a sensor package with various sensors therein. In addition, the wearable device 1002 can include various sensors therein, such as those described with respect to the sensor package of the hearing assistance device 200. Similarly, the external visual display device 504 can include various sensors therein, such as IMUs, accelerometers, optical sensors, temperature sensors, and the like. In various embodiments herein, data from one or more of these sources can be used to determine, estimate or calculate a body position and/or a posture of the subject. Whether or not the subject is in the correct position and/or posture for a particular exercise can be a useful additional point of feedback to provide to the subject. In addition, information regarding the position and/or posture of the subject can be provided to a care provider (such as 516 in FIG. 5), such as a care provider that is located remotely from the subject.

Estimating, determining or calculating body position and/or posture can be performed in various ways. In some embodiments, estimating a position and/or posture of the subject's body can include evaluating data from at least one sensor other than the first IMU. In some embodiments, estimating a posture of the subject's body can include matching data from the first IMU against a set of predetermined data patterns (including, but not limited to, templates) representing body postures using a pattern matching algorithm. In some embodiments, estimating a posture of the subject's body includes matching data from the first IMU and at least one sensor other than the first IMU against a set of predetermined data patterns representing body postures using a pattern matching algorithm. Pattern matching algorithms used herein can include, but are not limited to, least squares algorithms, cluster-based pattern recognition, template matching algorithms, machine learning algorithms and the like.

Table 1 below shows one example of data patterns associated with different specific positions/postures.

**TABLE 1**

| **Position/Posture** | **IMU / Accelerometer Data (Fixed Relative to Head)** | **Heart Rate** | **Blood Pressure** | **Weight on Feet** |
|---|---|---|---|---|
| Standing | Head Substantially Aligned with Gravity | Elevated Over Normal Resting for Subject | Elevated Over Normal Resting for Subj ect | Full Weight of Individual |
| Sitting | Head Substantially Aligned with Gravity to Head 10 Degrees or More Elevated with Respect to Perpendicular to Gravity | Normal Resting to Slightly Elevated Above Resting | Normal Resting to Slightly Elevated Above Resting | Weight Minimal or Nonexistent |
| Supine | Head Substantially Perpendicular to Gravity with Face Away from Gravitational Pull | Normal Resting to Slightly Elevated Above Resting | Normal Resting to Slightly Elevated Above Resting | Weight Minimal or Nonexistent |
| Prone | Head Substantially Perpendicular to Gravity with Face Away from Gravitational Pull | Normal Resting to Slightly Elevated Above Resting | Normal Resting to Slightly Elevated Above Resting | Weight Minimal or Non-Existent |
| Lying on Side | Head Substantially Perpendicular to Gravity with Face Perpendicular to Direction of Gravitational Pull | Normal Resting to Slightly Elevated Above Resting | Normal Resting to Slightly Elevated Above Resting | Weight Minimal or Non-Existent |

While Table 1 above shows one example of data patterns associated with different specific positions/postures, it will be appreciated that data from additional types of sensors can also be used to determine the posture or position of a subject's body. In addition, patterns can be developed that are specific for a particular patient and/or specific for a particular class of patients.

Information regarding position/posture of the patient can form part of visual feedback provided to the subject. For example, if a particular exercise requires the subject to lie down, and the system and/or hearing assistance device estimates that the patient is not lying down, the system can provide a notification to the user, such as through the display screen 806 and/or auditory feedback, directing them to lie down. Similarly, if a particular exercise requires the subject be standing or sitting and the system and/or hearing assistance device estimates that the patient is not standing or sitting, the system can provide a notification to the user, such as through the display screen 806 and/or auditory feedback, directing them to sit or stand. In some embodiments, if the subject is non-responsive (e.g., does not follow directions to sit or stand when the system estimates that they are lying down), the system and/or hearing assistance device can issue a warning. In some cases, the warning can be transmitted to a care provider 516 or a designated emergency recipient.

### Methods

Various methods are included herein. In some embodiments, methods of providing physical or vestibular therapy and/or exercises to a subject are included herein. In some embodiments, method steps described can be executed as a series of operations by devices described herein.

In an embodiment, a method of providing physical or vestibular therapy to a subject is included. The method can include prompting (visually or using an auditory prompt) the subject to move in a predetermined direction by a predetermined amount as part of a first predetermined movement. The first predetermined movement can be part of a vestibular training regimen or exercise routine. Vestibular training regimens and/or exercise routines herein can include a plurality of individual predetermined movements. The method can also include tracking movement of the subject using a first IMU disposed in a fixed position relative to the subject's head. The method can also include generating feedback data by comparing the tracked movement of the subject against the predetermined direction and amount of the first predetermined movement. The method can also include generating a feedback image using the feedback data.

In some embodiments, the method can further include tracking movement of the subject with a second IMU disposed in a fixed position relative to the subject's head, wherein the second IMU is spaced apart from the first IMU by a distance of at least three centimeters. In some embodiments, the first IMU and the second IMU are disposed on opposing lateral sides of the subject's head. In some embodiments, the first IMU and the second IMU are disposed within opposing ear canals of the subject. In some embodiments, the first IMU and the second three axis accelerometer are disposed on or in opposing ears of the subject. In some embodiments, the first IMU is mounted in a first hearing assistance device and the second IMU is mounted in a second hearing assistance device.

In some embodiments of the method, the second hearing assistance device wirelessly transmits data derived from the second IMU to the first hearing assistance device. In some embodiments, the first hearing assistance device wirelessly transmits data derived from the first IMU and the second IMU to an external visual display device. In some embodiments the hearing assistance devices duty cycle the transmission of feedback data in order to preserve resources (e.g., battery capacity). For example, in some embodiments, the system transmits data from the first hearing assistance device to the external visual display device (or a separate device) in a first time period, then transmits data from the second hearing assistance device to the external visual display device (or a separate device) in a second time period, and switches back and forth. In some embodiments, the system selects the hearing assistance device with the most remaining battery capacity for use in transmissions to and from the external visual display device and/or other separate devices.

In some embodiments of the method, the feedback image including a visual representation of the first predetermined movement and a visual representation of the tracked movement. In some embodiments, the visual representation of the first predetermined movement comprises a silhouette of a head. In some embodiments, the visual representation of the first predetermined movement comprises an image of the subject's head. In some embodiments, the visual representation of the tracked movement comprises a silhouette of a head. In some embodiments, the visual representation of the tracked movement comprises an image of the patient's head. In some embodiments, the visual representation of the tracked movement includes other parts of the body such as the arms, hands, shoulders, torso, hips, legs, knees, feet, and the like.

In some embodiments, generating feedback data by comparing the tracked movement of the subject against the predetermined direction and amount comprises assigning a score based on the feedback data. In some embodiments, statistics regarding successful and unsuccessful repetitions can be stored and reported on, such as part of feedback data. In some embodiments, the method can include providing a visual prompt to the subject for continued movement if the feedback data exceeds a threshold amount of deviation between the tracked movement of the subject against the first predetermined movement. In some embodiments, the visual representation of the predetermined movement changes based on the feedback data. In some embodiments, the visual representation of the predetermined movement changes color based on the feedback data.

In some embodiments, feedback data (including generation or and/or transmission of the same) may to stopped or paused between exercises (different types or repetitions). In some embodiments, feedback data (including generation or and/or transmission of the same) may to stopped or paused if a device is used for another purpose. For example, feedback data may be stopped or paused if the user receives a call on a smart phone serving as a feedback devices. Stoppage or pausing can provide various benefits including, but not limited to, conserving resources as well as resetting sensors that may drift over time, such as a gyroscope.

In some embodiments, the method can further include sending the feedback data to a remote system user at a remote site, receiving auditory feedback from the remote system user, and presenting the auditory feedback to the subject. In some embodiments, presenting the auditory feedback to the subject can be performed with a hearing assistance device. In some embodiments, presenting the auditory feedback to the subject is performed with a hearing assistance device and the auditory feedback is configured to be presented to the subject as spatially originating from a direction of an end point of the first predetermined movement.

In some embodiments, the method can further include estimating a position of the subject's body. In some embodiments, estimating a position of the subject's body includes estimating a posture of the subject's body. In some embodiments, estimating a posture of the subject's body includes evaluating data from at least one sensor other than the first IMU. In some embodiments, estimating a posture of the subject's body includes matching data from the first IMU against a set of predetermined data patterns representing body postures using a pattern matching algorithm. In some embodiments, estimating a posture of the subject's body includes matching data from the first IMU and at least one sensor other than the first IMU against a set of predetermined data patterns representing body postures using a pattern matching algorithm.

In some embodiments, a method of providing vestibular therapy to a subject is included herein that includes prompting the subject to move in a predetermined direction as part of a first predetermined movement. The method can further include tracking movement of the subject using a IMU positioned in a fixed position relative to their head. The method can further include generating a feedback image including a visual representation of the first predetermined movement and a visual representation of the tracked movement.

Alternatively, or in addition, the system and/or hearing assistance device can generate an electrical signal output indicating success or lack of success of each or a series of specified bodily actions. The signal output can be transmitted from the hearing assistance device to an external device, such as a wrist-worn electronic device (e.g., a smart watch), smartphone, tablet, laptop, internet gateway, or other electronic device. In response to the signal output, the external device can generate a visual output indicating success or lack of success of each or a series of specified actions. Corrective feedback can also be generated by the external device. The output produced by the external device can be one or a combination of a visual, auditory (e.g., sounds and/or speech) or tactile output. The signal output can also be stored in a memory internal to or external of the hearing assistance device (e.g., a memory of an external device).

In some embodiments, the stored signal output, which can include other data associated with the predetermined maneuver, physical therapy or exercise routine, can be transmitted from the hearing assistance device and/or external device to a remote server. The associated data can include one or more of the name of the maneuver/therapy/routine, time and date of execution, statistics regarding successful and unsuccessful performances, and wearer ID information, for example. The remote server can store such data acquired from a multiplicity of wearers.

### Sensors

According to various embodiments, hearing assistance devices herein can include a sensor package or arrangement configured to sense various aspects such as the movement of the wearer during each of the body actions required to implement a predetermined corrective or therapeutic maneuver, physical therapy or exercise routine. The sensor package can comprise one or a multiplicity of sensors, such one or more of an inertial measurement unit (IMU), accelerometer, gyroscope, barometer, magnetometer, microphone, optical sensor, camera, electroencephalography (EEG) and eye movement sensor (e.g., electrooculogram (EOG) sensor). In some embodiments, the sensor package can comprise one or more additional sensors that are external of the hearing assistance device. The one or more additional sensors can comprise one or more of an IMU, accelerometer, gyroscope, barometer, magnetometer, an acoustic sensor, eye motion tracker, EEG or myographic potential electrode (e.g., EMG), heart rate monitor, and pulse oximeter. For example, the one or more additional sensors can include a wrist-worn or ankle-worn sensor package, or a sensor package supported by a chest strap. In some embodiments, the additional sensor can include a camera, such as one embedded within a device such as glasses frames.

The sensor package of a hearing assistance device is configured to sense movement of the wearer as he or she executes each action of a predetermined corrective or therapeutic maneuver, physical therapy or exercise routine. Data produced by the sensor package is operated on by a processor of the hearing assistance device to determine if a specified action was successfully or unsuccessfully executed by the wearer.

According to various embodiments, the sensor package can include one or more of an IMU, and accelerometer (3, 6, or 9 axis), a gyroscope, a barometer, a magnetometer, an eye movement sensor, a pressure sensor, an acoustic sensor, a heart rate sensor, an electrical signal sensor (such as an EEG, EMG or ECG sensor), a temperature sensor, a blood pressure sensor, an oxygen saturation sensor, an optical sensor, and the like. As used herein the term "inertial measurement unit" or "IMU" shall refer to an electronic device that can generate signals related to a body's specific force and/or angular rate. IMUs herein can include one or more of an accelerometer (3, 6, or 9 axis) to detect linear acceleration and a gyroscope to detect rotational rate. In some embodiments, an IMU can also include a magnetometer to detect a magnetic field. The eye movement sensor may be, for example, an electrooculographic (EOG) sensor, such as an EOG sensor disclosed in commonly owned U.S. Patent No. 9,167,356, which is incorporated herein by reference. The pressure sensor can be, for example, a MEMS-based pressure sensor, a piezo-resistive pressure sensor, a flexion sensor, a strain sensor, a diaphragm-type sensor and the like. The temperature sensor can be, for example, a thermistor (thermally sensitive resistor), a resistance temperature detector, a thermocouple, a semiconductor-based sensor, an infrared sensor, or the like. The blood pressure sensor can be, for example, a pressure sensor. The heart rate sensor can be, for example, an electrical signal sensor, an acoustic sensor, a pressure sensor, an infrared sensor, an optical sensor, or the like. The oxygen saturation sensor can be, for example, an optical sensor, an infrared sensor, or the like. The electrical signal sensor can include two or more electrodes and can include circuitry to sense and record electrical signals including sensed electrical potentials and the magnitude thereof (according to Ohm's law where V = IR) as well as measure impedance from an applied electrical potential.

The sensor package can include one or more sensors that are external to the hearing assistance device. In addition to the external sensors discussed hereinabove, the sensor package can comprise a network of body sensors (such as those listed above) that sense movement of a multiplicity of body parts (e.g., arms, legs, torso).

### Virtual Audio Interfaces

In some embodiments, a virtual audio interface can be used to provide auditory feedback to a subject in addition to visual feedback as described elsewhere herein. The virtual audio interface can be configured to synthesize three-dimensional (3-D) audio that guides the wearer in performing specific physical movements of a predetermined corrective or therapeutic maneuver, physical therapy or exercise routine.

According to some embodiments, the virtual audio interface can generate audio cues comprising spatialized 3-D virtual sound emanating from virtual spatial locations that serve as targets for guiding wearer movement. The wearer can execute a series of body movements in a direction and/or extent indicated by a sequence of virtual sound 5 targets. The sound generated at the virtual spatial locations can be any broadband sound, such as complex tones, noise bursts, human speech, music, etc. or a combination of these and other types of sound. In various embodiments, the virtual audio interface is configured to generate binaural or monaural sounds, alone or in combination with spatialized 3-D virtual sounds. The binaural and monaural sounds can be any of those listed above including single-frequency tones.

In other embodiments, the virtual audio interface is configured to generate human
speech that guides the wearer in performing specific physical movements of a predetermined corrective or therapeutic maneuver, physical therapy or exercise routine. The speech can be synthesized speech or a pre-recording of real speech. In embodiments that employ a single hearing assistance device (for one ear), for example, the virtual audio interface generates monaural sound in the form of speech, which can be accompanied by other sounds, such as single or multi-frequency tones, noise bursts or music. In embodiments that employ two hearing assistance devices (one device for each ear), the virtual audio interface can generate monaural or binaural sound in the form of speech, which can be accompanied by other sounds, such as single or multi-frequency tones, noise bursts or music. The virtual audio interface can display (play back) spoken instructions to guide the wearer though specific physical movements of a predetermined corrective or therapeutic maneuver, physical therapy or exercise routine. Further aspects of virtual audio interfaces are described in commonly owned U.S. Pat. Appl. No. 15/589,298, titled "Hearing Assistance Device Incorporating Virtual Audio Interface for Therapy Guidance", the content of which is herein incorporated by reference in its entirety.

### Exercise Movements

In accordance with various embodiments herein, hearing assistance devices that
can be configured to guide the wearer of a hearing assistance device through a prescribed series of body movements or actions in accordance with a predetermined corrective or therapeutic maneuver, physical therapy or exercise routine. A maneuver, physical therapy or exercise routine involves a prescribed series of body movements or actions that can be implemented by the wearer of a hearing assistance device in an attempt to correct or treat a physiologic disorder or execute a physical fitness routine. Exercises (or routines or maneuvers herein) can include, but are not limited to, habituation exercises, gaze stabilization exercises, and balance training exercises. Exercises can include a series of actions including one or more of turning their head in a specified direction by a specified amount, moving their head in a specific direction by a specified amount, focusing their gaze on a stationary or moving point, assuming different postures, etc.

Guidance and/or feedback herein can include auditory guidance, visual guidance, or auditory and visual guidance. Audio guidance can include any one or a combination of different sounds, such as tones, noise bursts, human speech, animal/natural sounds, synthesized sounds, and music, among other sounds.

For example, the virtual audio interface can display spoken words that instruct the wearer to assume a specific position, such as lying down, standing or sitting up. A spoken instruction can be displayed that requests the wearer to move a specific body part in a particular manner. For example, the wearer can be instructed to turn his or her head by approximately 45° to the right (e.g., "turn your head so your nose is pointing 45° to the right"). A synthesized 3-D virtual audio target can be generated at the specified location relative to the wearer's current head position. In response, the wearer moves his or her head in the specified direction indicated by the audio target.

In some embodiments, an exercise can be turned into a game wherein control elements/inputs for the game can be linked to sensed movements/actions of the subject while performing exercises including, but not limited to, movement or rotation of the head, directional gaze of the eyes, etc. Control elements can include, but are not limited to, virtual button presses/inputs, directional inputs, and the like.

BPPV occurs because of displaced otoconia, which are small crystals of calcium carbonate (also referred to as "otoliths" or "canaliths") that are normally attached to the otolithic membrane in the utricle of the inner ear. Because of trauma, infection, or even simple aging, canaliths can detach from the utricle and collect within the semicircular canals. Head movements shift the detached canaliths and stimulate sensitive nerve hairs to send false signals to the brain, causing dizziness and other symptoms. The goal of the canalith repositioning procedure (CRP), a form of vestibular rehabilitation therapy, is to move the displaced canaliths to stop these false signals and the debilitating symptoms they can cause.

In some embodiments, the exercise movement can include the Epley maneuver. For example, to address an issue on the left side (e.g., BPPV determined to be caused by issues with the left ear), the steps in Table 2 can be followed.

**TABLE 2**

| **STEP #** | **DESCRIPTION** |
|---|---|
| STEP 1 | Start sitting up on a bed, with your legs flat on the bed in front of you. Turn your head 45 degrees to the left. |
| STEP 2 | Lie down, keeping your head turned to the left. Wait 30 seconds. |
| STEP 3 | Turn your head to the right 90 degrees, until it's facing 45 degrees to your right side. Wait 30 seconds. |
| STEP 4 | Roll over onto your right side before sitting up. |

As another example, to address an issue on the right side (e.g., BPPV determined to be caused by issues with the right ear), the steps in Table 3 can be followed.

**TABLE 3**

| **STEP #** | **DESCRIPTION** |
|---|---|
| STEP 1 | Start sitting up on a bed, with your legs flat on the bed in front of you. Turn your head 45 degrees to the right. |
| STEP 2 | Lie down, keeping your head turned to the right. Wait 30 seconds. |
| STEP 3 | Turn your head to the left 90 degrees, until it's facing 45 degrees to your left side. Wait 30 seconds. |
| STEP 4 | Roll over onto your left side before sitting up. |

In some embodiments, exercises herein can include Brandt-Daroff exercises. The procedure involves sitting on the edge of a bed, moving into a side-lying position until the vertigo ends, returning to the sitting position for a fixed interval, and then moving into a side-lying position on the opposite side, and so on. These exercises can be repeated in multiple sets throughout each day until two days after vertigo has not been experienced.

Depending on the vestibular-related problem(s) identified, three principal methods of exercise can be prescribed: 1) habituation, 2) gaze stabilization, and/or 3) balance training. Habituation exercises can be used to treat symptoms of dizziness that are produced because of self-motion and/or produced because of visual stimuli. Habituation exercise is indicated for patients who report increased dizziness when they move around, especially when they make quick head movements, or when they change positions like when they bend over or look up to reach above their heads. Also, habituation exercise is appropriate for patients who report increased dizziness in visually stimulating environments, like shopping malls and grocery stores, when watching action movies or T.V., and/or when walking over patterned surfaces or shiny floors.

Habituation exercise is not suited for dizziness symptoms that are spontaneous in nature and do not worsen because of head motion or visual stimuli. The goal of habituation exercise is to reduce the dizziness through repeated exposure to specific movements or visual stimuli that provoke patients' dizziness. These exercises are designed to mildly, or at the most moderately, provoke the patients' symptoms of dizziness. The increase in symptoms should only be temporary, and before continuing onto other exercises or tasks the symptoms should return completely to the baseline level. Over time and with good compliance and perseverance, the intensity of the patient's dizziness will decrease as the brain learns to compensate for the abnormal signals it is receiving from the inner ear, eighth cranial nerve (CN-VIII), central lesion, or the like.

Gaze stabilization exercises can be used to improve control of eye movements, so vision can be clear during head movement. These exercises are appropriate for patients who report problems seeing clearly because their visual world appears to bounce or jump around, such as when reading or when trying to identify objects in the environment, especially when moving about.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains. All publications and patent applications are herein incorporated by reference to the same extent as if each individual publication or patent application was specifically and individually indicated by reference.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

## Claims

1. A method of providing visual feedback to a subject (602) undergoing physical therapy or vestibular movement comprising:
providing a visual and/or auditory notification to the subject (602) for moving in a predetermined direction by a predetermined amount as part of a first predetermined movement;
tracking movement of the subject (602) using a first inertia measurement unit 'IMU' mounted in a first hearing assistance device and disposed in a fixed position relative to the subject's head;
generating feedback data by comparing the tracked movement of the subject (602) against the predetermined direction and amount of the first predetermined movement; and
generating a feedback image using the feedback data.

2. The method of claim 1, further comprising tracking movement of the subject (602) using a second IMU disposed in a fixed position relative to the subject's head, wherein at least one of the first IMU and the second IMU are disposed within opposing ear canals of the subject.

3. The method of claim 1, further comprising tracking movement of the subject (602) using a second IMU disposed in a fixed position relative to the subject's head, wherein the first IMU and the second IMU are disposed on or about opposing ears of the subject.

4. The method of claim 1, further comprising tracking movement of the subject (602) using a second IMU disposed in a fixed position relative to the subject's head, wherein the second IMU is mounted in a second hearing assistance device, wherein the first hearing assistance device wirelessly transmits data derived from the first IMU to an external visual display device and the second hearing assistance device wirelessly transmits data derived from the second IMU to the external visual display device.

5. The method of any of claims 1-4, the feedback image including a visual representation of the first predetermined movement and a visual representation of the tracked movement.

6. The method of any of claims 1-5, wherein generating feedback data by comparing the tracked movement of the subject against the predetermined direction and amount comprises assigning a score based on the feedback data.

7. The method of any of claims 1-6, further comprising providing a visual prompt to the subject for continued movement if the feedback data exceeds a threshold amount of deviation between the tracked movement of the subject against the first predetermined movement.

8. The method of any of claims 1-7, further comprising
sending the feedback data to a remote system user at a remote site;
receiving feedback from the remote system user; and
presenting the feedback to the subject.

9. The method of claim 8, wherein presenting the feedback to the subject is performed with a hearing assistance device.

10. The method of any of claims 1-9, further comprising estimating a position of the subject's body.

11. The method of claim 10, wherein estimating a position of the subject's body includes estimating a posture of the subject's body,
wherein estimating a posture of the subject's body includes matching data from the first IMU against a set of predetermined data patterns representing body postures using a pattern matching algorithm.

12. A hearing assistance device (200) comprising:
a first control circuit (322);
a first inertia measurement unit 'IMU' in electrical communication with the first control circuit (322), wherein the first IMU is mounted in the hearing assistance device (200) and disposed in a fixed position relative to a head of a subject (602) wearing the hearing assistance device (200);
a first microphone (306) in electrical communication with the first control circuit (322);
a first electroacoustic transducer for generating sound in electrical communication with the first control circuit (322);
a first power supply (314) circuit in electrical communication with the first control circuit (322);
wherein the first control circuit (322) is configured to track movement of the subject (602) using the first IMU, and
**characterised in that**
the first control circuit (322) is configured to generate feedback data reflecting a comparison of the tracked movement of the subject (602) against a predetermined direction and amount of movement as part of a first predetermined movement.

13. The hearing assistance device (200) of claim 12, the hearing assistance (200) device further comprising a first communications circuit in electrical communication with the first control circuit (322), the hearing assistance device configured to wirelessly transmit feedback data from the first communications circuit to an external visual display device.

14. The hearing assistance device (200) of any of claims 12-13, further comprising:
a second control circuit;
a second IMU in electrical communication with the second control circuit, wherein the second IMU is disposed in a fixed position relative to the head of a subject wearing the hearing assistance device;
a second microphone in electrical communication with the second control circuit;
a second electroacoustic transducer for generating sound in electrical communication with the second control circuit;
a second power supply circuit in electrical communication with the second control circuit;
a second communications circuit in electrical communication with the second control circuit;
wherein the second control circuit is configured to
track movement of the subject using the second IMU, and send data from the second IMU to the first control circuit; or
track movement of the subject using the second IMU, and generate feedback data reflecting a comparison of the tracked movement of the subject against a predetermined direction and amount of movement using data from the second IMU.

15. The hearing assistance device of claim 14, wherein the first control circuit is configured to generate feedback data reflecting a comparison of the tracked movement of the subject against a predetermined direction and amount of movement using data from both the first IMU and the second IMU.

## Patentansprüche

1. Verfahren zum Bereitstellen visueller Rückmeldung an ein Subjekt (602), das sich physischer Therapie oder vestibulärer Bewegung unterzieht, umfassend:
Bereitstellen einer visuellen und/oder hörbaren Benachrichtigung an das Subjekt (602), um sich als Teil einer ersten vorgegebenen Bewegung um eine vorgegebene Menge in eine vorgegebene Richtung zu bewegen;
Verfolgen von Bewegung des Subjekts (602) unter Verwendung einer ersten Trägheitsmesseinheit 'IMU', die in einer ersten Hörhilfevorrichtung montiert ist und in einer fixierten Position relativ zu dem Kopf des Subjekts angeordnet ist;
Erzeugen von Rückmeldungsdaten, indem die nachverfolgte Bewegung des Subjekts (602) mit der vorgegebenen Richtung und Menge der ersten vorgegebenen Bewegung verglichen wird; und
Erzeugen eines Rückmeldungsbilds unter Verwendung der Rückmeldungsdaten.

2. Verfahren nach Anspruch 1, das weiter Nachverfolgen von Bewegung des Subjekts (602) unter Verwendung einer zweiten IMU umfasst, die in einer fixierten Position relativ zu dem Kopf des Subjekts angeordnet ist, wobei mindestens eine der ersten IMU und der zweiten IMU innerhalb entgegengesetzter Ohrkanäle des Subjekts angeordnet ist.

3. Verfahren nach Anspruch 1, das weiter Nachverfolgen von Bewegung des Subjekts (602) unter Verwendung einer zweiten IMU umfasst, die in einer fixierten Position relativ zu dem Kopf des Subjekts angeordnet ist, wobei die erste IMU und die zweite IMU auf oder um entgegengesetzte Ohren des Subjekts angeordnet sind.

4. Verfahren nach Anspruch 1, das weiter Nachverfolgen von Bewegung des Subjekts (602) unter Verwendung einer zweiten IMU umfasst, die in einer fixierten Position relativ zu dem Kopf des Subjekts angeordnet ist, wobei die zweite IMU in einer zweiten Hörhilfevorrichtung montiert ist, wobei die erste Hörhilfevorrichtung Daten, die von der ersten IMU abgeleitet werden, drahtlos an eine externe visuelle Anzeigevorrichtung überträgt, und die zweite Hörhilfevorrichtung Daten, die von der zweiten IMU abgeleitet werden, drahtlos an die externe visuelle Anzeigevorrichtung überträgt.

5. Verfahren nach einem der Ansprüche 1-4, wobei das Rückmeldungsbild eine visuelle Darstellung der ersten vorgegebenen Bewegung und eine visuelle Darstellung der nachverfolgten Bewegung beinhaltet.

6. Verfahren nach einem der Ansprüche 1-5, wobei Erzeugen von Rückmeldungsdaten, indem die nachverfolgte Bewegung des Subjekts mit der vorgegebenen Richtung und Menge verglichen wird, Zuweisen eines Werts umfasst, der auf den Rückmeldungsdaten basiert.

7. Verfahren nach einem der Ansprüche 1-6, das weiter Bereitstellen einer visuellen Aufforderung an das Subjekt für fortgesetzte Bewegung bereitstellt, falls die Rückmeldungsdaten eine Schwellenabweichungsmenge zwischen der nachverfolgten Bewegung des Subjekts und der ersten vorgegebenen Bewegung übersteigt.

8. Verfahren nach einem der Ansprüche 1-7, weiter umfassend
Senden der Rückmeldungsdaten an einen Fernsystemnutzer an einer ferngelegenen Stelle;
Empfangen von Rückmeldung von dem Fernsystemnutzer; und
Darstellen der Rückmeldung für das Subjekt.

9. Verfahren nach Anspruch 8, wobei Darstellen der Rückmeldung für das Subjekt mit einer Hörhilfevorrichtung durchgeführt wird.

10. Verfahren nach einem der Ansprüche 1-9, das weiter Schätzen einer Position des Körpers des Subjekts umfasst.

11. Verfahren nach Anspruch 10, wobei Schätzen einer Position des Körpers des Subjekts Schätzen einer Körperhaltung des Subjekts beinhaltet,
wobei Schätzen einer Körperhaltung des Subjekts Abgleichen von Daten von der ersten IMU mit einem Satz vorgegebener Datenstrukturen, die Körperhaltungen darstellen, unter Verwendung eines Strukturabgleichalgorithmus beinhaltet.

12. Hörhilfevorrichtung (200), umfassend:
eine erste Steuerschaltung (322);
eine erste Trägheitsmesseinheit 'IMU' in elektrischer Kommunikation mit der ersten Steuerschaltung (322), wobei die erste IMU in der Hörhilfevorrichtung (200) montiert ist und in einer fixierten Position relativ zu einem Kopf eines Subjekts (602) angeordnet ist, das die Hörhilfevorrichtung (200) trägt;
ein erstes Mikrofon (306) in elektrischer Kommunikation mit der ersten Steuerschaltung (322);
einen ersten elektroakustischen Wandler zum Erzeugen von Schall in elektrischer Kommunikation mit der ersten Steuerschaltung (322);
eine erste Stromzufuhrschaltung (314) in elektrischer Kommunikation mit der ersten Steuerschaltung (322);
wobei die erste Steuerschaltung (322) dazu konfiguriert ist, Bewegung des Subjekts (602) unter Verwendung der ersten IMU nachzuverfolgen, und
**dadurch gekennzeichnet, dass**
die erste Steuerschaltung (322) dazu konfiguriert ist, Rückmeldungsdaten zu erzeugen, die einen Vergleich der nachverfolgten Bewegung des Subjekts (602) mit einer vorgegebenen Bewegungsrichtung und -menge als Teil einer ersten vorgegebenen Bewegung widerspiegeln.

13. Hörhilfevorrichtung (200) nach Anspruch 12, wobei die Hörhilfevorrichtung (200) weiter eine erste Kommunikationsschaltung in elektrischer Kommunikation mit der ersten Steuerschaltung (322) umfasst, wobei die Hörhilfevorrichtung dazu konfiguriert ist, drahtlos Rückmeldungsdaten von der ersten Kommunikationsschaltung an eine externe visuelle Anzeigevorrichtung zu übertragen.

14. Hörhilfevorrichtung (200) nach einem der Ansprüche 12-13, weiter umfassend:
eine zweite Steuerschaltung;
eine zweite IMU in elektrischer Kommunikation mit der zweiten Steuerschaltung, wobei die zweite IMU in einer fixierten Position relativ zu dem Kopf eines Subjekts angeordnet ist, das die Hörhilfevorrichtung trägt;
ein zweites Mikrofon in elektrischer Kommunikation mit der zweiten Steuerschaltung;
einen zweiten elektroakustischen Wandler zum Erzeugen von Schall in elektrischer Kommunikation mit der zweiten Steuerschaltung;
eine zweite Stromzufuhrschaltung in elektrischer Kommunikation mit der zweiten Steuerschaltung;
eine zweite Kommunikationsschaltung in elektrischer Kommunikation mit der zweiten Steuerschaltung;
wobei die zweite Steuerschaltung konfiguriert ist zum
Nachverfolgen von Bewegung des Subjekts unter Verwendung der zweiten IMU und Senden von Daten von der zweiten IMU an die erste Steuerschaltung; oder
Nachverfolgen von Bewegung des Subjekts unter Verwendung der zweiten IMU und Erzeugen von Rückmeldungsdaten, die einen Vergleich der nachverfolgten Bewegung des Subjekts mit einer vorgegebenen Bewegungsrichtung und -menge widerspiegeln, unter Verwendung von Daten der zweiten IMU.

15. Hörhilfevorrichtung nach Anspruch 14,
wobei die erste Steuerschaltung dazu konfiguriert ist, Rückmeldungsdaten zu erzeugen, die einen Vergleich der nachverfolgten Bewegung des Subjekts mit einer vorgegebenen Bewegungsrichtung und -menge widerspiegeln, unter Verwendung von Daten sowohl der ersten IMU als auch der zweiten IMU.

## Revendications

1. Procédé de fourniture d'une rétroaction visuelle à un sujet (602) soumis à une thérapie physique ou à un mouvement vestibulaire comprenant :
la fourniture d'une notification visuelle et/ou auditive au sujet (602) pour le déplacement dans une direction prédéterminée d'une quantité prédéterminée dans le cadre d'un premier mouvement prédéterminé ;
le suivi du mouvement du sujet (602) en utilisant une première unité de mesure de l'inertie (IMU) montée dans un premier dispositif d'aide auditive et disposée dans une position fixe par rapport à la tête du sujet ;
la génération de données de rétroaction en comparant le mouvement suivi du sujet (602) avec la direction et la quantité prédéterminées du premier mouvement prédéterminé ; et
la génération d'une image de rétroaction en utilisant les données de rétroaction.

2. Procédé selon la revendication 1, comprenant en outre le suivi du mouvement du sujet (602) en utilisant une seconde IMU disposée dans une position fixe par rapport à la tête du sujet, dans lequel au moins l'une de la première IMU et de la seconde IMU est disposée à l'intérieur des conduits auditifs opposés du sujet.

3. Procédé selon la revendication 1, comprenant en outre le suivi du mouvement du sujet (602) en utilisant une seconde IMU disposée dans une position fixe par rapport à la tête du sujet, dans lequel la première IMU et la seconde IMU sont disposées sur ou autour d'oreilles opposées du sujet.

4. Procédé selon la revendication 1, comprenant en outre le suivi du mouvement du sujet (602) en utilisant une seconde IMU disposée dans une position fixe par rapport à la tête du sujet, dans lequel la seconde IMU est montée dans un second dispositif d'aide auditive, dans lequel le premier dispositif d'aide auditive transmet sans fil des données dérivées de la première IMU à un dispositif d'affichage visuel externe et le second dispositif d'aide auditive transmet sans fil des données dérivées de la seconde IMU au dispositif d'affichage visuel externe.

5. Procédé selon l'une quelconque des revendications 1-4, l'image de rétroaction incluant une représentation visuelle du premier mouvement prédéterminé et une représentation visuelle du mouvement suivi.

6. Procédé selon l'une quelconque des revendications 1-5, dans lequel la génération de données de rétroaction en comparant le mouvement suivi du sujet avec la direction et la quantité prédéterminées comprend l'attribution d'un score sur la base des données de rétroaction.

7. Procédé selon l'une quelconque des revendications 1-6, comprenant en outre la fourniture d'une invite visuelle au sujet pour qu'il poursuive son mouvement si les données de rétroaction dépassent une quantité d'écart seuil entre le mouvement suivi du sujet et le premier mouvement prédéterminé.

8. Procédé selon l'une quelconque des revendications 1-7, comprenant en outre
l'envoi des données de rétroaction à un utilisateur du système à distance sur un site distant ;
la réception d'une rétroaction de la part de l'utilisateur du système à distance ; et
la présentation de la rétroaction au sujet.

9. Procédé selon la revendication 8, dans lequel la présentation de la rétroaction au sujet est effectuée avec un dispositif d'aide auditive.

10. Procédé selon l'une quelconque des revendications 1-9, comprenant en outre l'estimation d'une position du corps du sujet.

11. Procédé selon la revendication 10, dans lequel l'estimation d'une position du corps du sujet inclut l'estimation d'une posture du corps du sujet,
dans lequel l'estimation d'une posture du corps du sujet inclut la mise en correspondance de données provenant de la première IMU avec un ensemble de modèles de données prédéterminés représentant des postures corporelles en utilisant un algorithme de mise en correspondance de modèles.

12. Dispositif d'aide auditive (200) comprenant :
un premier circuit de commande (322) ;
une première unité de mesure de l'inertie (IMU) en communication électrique avec le premier circuit de commande (322), dans lequel la première IMU est montée dans le dispositif d'aide auditive (200) et disposée dans une position fixe par rapport à une tête d'un sujet (602) portant le dispositif d'aide auditive (200) ;
un premier microphone (306) en communication électrique avec le premier circuit de commande (322) ;
un premier transducteur électroacoustique pour générer un son en communication électrique avec le premier circuit de commande (322) ;
un premier circuit d'alimentation (314) en communication électrique avec le premier circuit de commande (322) ;
dans lequel le premier circuit de commande (322) est configuré pour suivre le mouvement du sujet (602) en utilisant la première IMU, et
**caractérisé en ce que**
le premier circuit de commande (322) est configuré pour générer des données de rétroaction reflétant une comparaison du mouvement suivi du sujet (602) avec une direction et une quantité de mouvement prédéterminées dans le cadre d'un premier mouvement prédéterminé.

13. Dispositif d'aide auditive (200) selon la revendication 12, le dispositif d'aide auditive (200) comprenant en outre un premier circuit de communication en communication électrique avec le premier circuit de commande (322), le dispositif d'aide auditive étant configuré pour transmettre sans fil des données de rétroaction du premier circuit de communication à un dispositif d'affichage visuel externe.

14. Dispositif d'aide auditive (200) selon l'une quelconque des revendications 12-13, comprenant en outre :
un second circuit de commande ;
une seconde IMU en communication électrique avec le second circuit de commande, dans lequel la seconde IMU est disposée dans une position fixe par rapport à la tête d'un sujet portant le dispositif d'aide auditive ;
un second microphone en communication électrique avec le second circuit de commande ;
un second transducteur électroacoustique pour générer un son en communication électrique avec le second circuit de commande ;
un second circuit d'alimentation en communication électrique avec le second circuit de commande ;
un second circuit de communication en communication électrique avec le second circuit de commande ;
dans lequel le second circuit de commande est configuré pour
suivre le mouvement du sujet en utilisant la seconde IMU, et envoyer des données provenant de la seconde IMU au premier circuit de commande ; ou
suivre le mouvement du sujet en utilisant la seconde IMU, et générer des données de rétroaction reflétant une comparaison du mouvement suivi du sujet avec une direction et une quantité de mouvement prédéterminées en utilisant des données provenant de la seconde IMU.

15. Dispositif d'aide auditive selon la revendication 14,
dans lequel le premier circuit de commande est configuré pour générer des données de rétroaction reflétant une comparaison du mouvement suivi du sujet avec une direction et une quantité de mouvement prédéterminées en utilisant des données provenant à la fois de la première IMU et de la seconde IMU.
